Europäisches Patentamt

European Patent Office

Office européen des brevets

(19)

(11) Publication number: **0 006 105**
**B1**

(12) **EUROPEAN PATENT SPECIFICATION**

(45) Date of publication of patent specification: **24.11.82**

(21) Application number: **79100410.4**

(22) Date of filing: **12.02.79**

(51) Int. Cl.³: **C 07 C 41/02,**
**C 07 C 41/03, B 01 J 23/02**

(54) **Barium oxide catalyzed ethoxylation.**

(30) Priority: **16.06.78 US 916421**

(43) Date of publication of application:
**09.01.80 Bulletin 80/1**

(45) Publication of the grant of the patent:
**24.11.82 Bulletin 82/47**

(84) Designated Contracting States:
**BE DE FR GB SE**

(56) References cited:
**CA - A - 558 226**
**CA - A - 558 227**
**Chemisches Zentralblatt, Volume 129, Nr. 45,**
**November 5, 1958, Berlin R. KUHN et al. "Zur**
**Methylierung von N-**
**Acetylglucosaminderivaten" pages 12 694 to 12**
**695**

(73) Proprietor: **CONOCO INC.**
**1000 South Pine Street P.O. Box 1267**
**Ponca City Oklahoma 74601 (US)**

(72) Inventor: **Yang, Kang**
**2501 Robin Road**
**Ponca City Oklahoma 74601 (US)**

(74) Representative: **Patentanwälte Dipl.-Ing. A.**
**Grünecker, Dr.-Ing. H. Kinkeldey, Dr.-Ing. W.**
**Stockmair,**
**Dr. rer. nat. K. Schumann, Dipl.-Ing. P. Jakob, Dr.**
**rer. nat. G. Bezold Maximilianstrasse 43**
**D-8000 München 22 (DE)**

Courier Press, Leamington Spa, England.

# 0 006 105

## Barium Oxide Catalyzed ethoxylation

This invention relates to the production of ethoxylated alcohols by reacting said alcohols with ethylene oxide. More particularly, this invention relates to the production of ethoxylated alcohols by reacting said alcohols in the presence of a catalyst, which is barium oxide, barium metal, barium hydroxide, or a barium hydroxide hydrate.

The general reaction of alcohols and ethylene oxide to form ethoxylated alcohols or ethylene oxide adducts, has long been known and practiced on a commercial scale. For example, these ethylene oxide adducts have been used as detergents and cleaning agents, domestic and industrial laundry detergents, detergent builders, polishes, sanitizers, and dry cleaning materials. Other users include the pulp and paper industry, and the fiber industry. These materials are especially adapted to these uses since they have functional properties such as wetting power, foaming, emulsifying and dispersing abilities as well as solubilization and detergent abilities to facilitate their use.

Much literature is available in the general area of ethoxylation of alcohols. Many references are also available relating to the catalytic ability of various materials, and the mechanism and kinetics of these reactions. For example, French Patent 1,365,945 teaches the use of compounds containing an active hydrogen atom reacted with ethylene oxide in the presence of alkali metal base. Acidic catalysts in general are also known. However, the ethoxylation of alcohols inevitably produces a distribution of various adducts. For example, in surfactant applications an adduct with too few ethylene oxide molecules is not effective because of poor solubility, while an adduct with too many ethylene oxide molecules is likewise undesirable because surface tension reduction per unit mass decreases drastically with increasing molecular weight. Thus it has long been essential to produce and use ethoxylates with as sharp a distribution in the desired mole adduct range (5 to 10) as possible. Acid catalyzed reactions such as that described above produce such ethoxylates, but these catalysts produce harmful side products such as dioxanes which must be separated and removed prior to use.

Russian Patent 523,074 teaches that alkali metals and various carbonates can be used to catalyze this reaction. The side product formation is these base catalyzed reactions is very low, but in base catalyzed reactions the adduct distribution is undesirably broad, such that a large proportion of the product obtained is not useful.

Representative of, but not exclusive of, the art in this area is U.S. Patent 3,328,467 which describes the use of zeolites and modified zeolites as catalysts in ethoxylation reactions. French 1,557,407 uses triethyloxonium fluoroborate to catalyze such reactions. Indeed, the art abounds with references to alkali metal hydroxides, such as sodium and potassium hydroxide, tertiary amines and sodium metal. German Offenlegungsschrift 2,639,564 teaches polyalkoxylation of active hydrogen compounds in the presence of sodium trifluoroborate or perchlorates of metals such as magnesium, calcium, manganese or zinc. U.S. Patent 3,969,417 uses tertiary oxonium salts as a catalyst. However, all these materials have the disadvantages described and set forth above.

US—PS 3 475 499 teaches the epoxidation of a mixture of normal alpha olefins by reaction with an organic hydroperoxide in the presence of a catalyst. The resulting 1,2-epoxides are reacted with water or ethylene glycol to form the corresponding glycols or glycol ethers. The latter reaction, which leads to specific monomer glycols and glycol ethers may be catalyzed by many acids or bases, e.g. barium hydroxide.

It would therefore be of great benefit to provide a catalyst which provides the low by-product levels of base catalysts, yet has the narrow distribution of the preferred mole adducts obtained from acid catalysts. Such a catalyst, which would promote the narrowing of the product distribution curve, would contribute significantly to the intrinsic value of the ethoxylates produced.

It is therefore an object of the present invention to provide a catalyst which will yield a narrow, high mole adduct distribution from the reaction of alcohols of all classes with ethylene oxide while providing low levels of undesirable by-products and unreacted free alcohols. Other objects will become apparent to those skilled in this art as the description proceeds.

It has now been discovered according to the instant invention that ethoxylation of all classes of alkanols can be carried out in the presence of barium oxide, hydrated barium hydroxide, barium hydroxide and barium metal, providing a narrow distribution of high mole ethylene oxide adducts while yielding a very low level of free alcohols and undesirable by-products.

Thus, the instant invention describes a method for the ethoxylation of alcohols comprising contacting said alcohol with ethylene oxide in the presence of catalyst, which is barium oxide, barium metal, barium hydroxide, or a barium hydroxide hydrate. The instant invention is carried out at temperatures of from 93°C to 260°C. Normally, the alcohols reacted under the process of the instant invention will contain from 4 to 20 carbon atoms but alcohols containing from 10 to 16 carbon atoms are those most used for commercial purposes. In a preferred embodiment of the mentioned method, the catalyst is barium oxide or hydrated barium hydroxide. Preferred as alcohol is a primary alcohol.

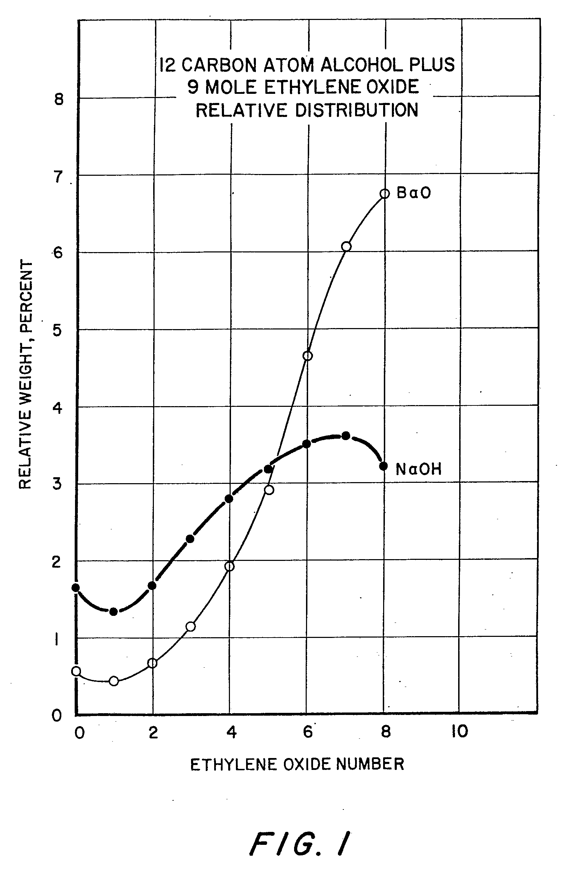

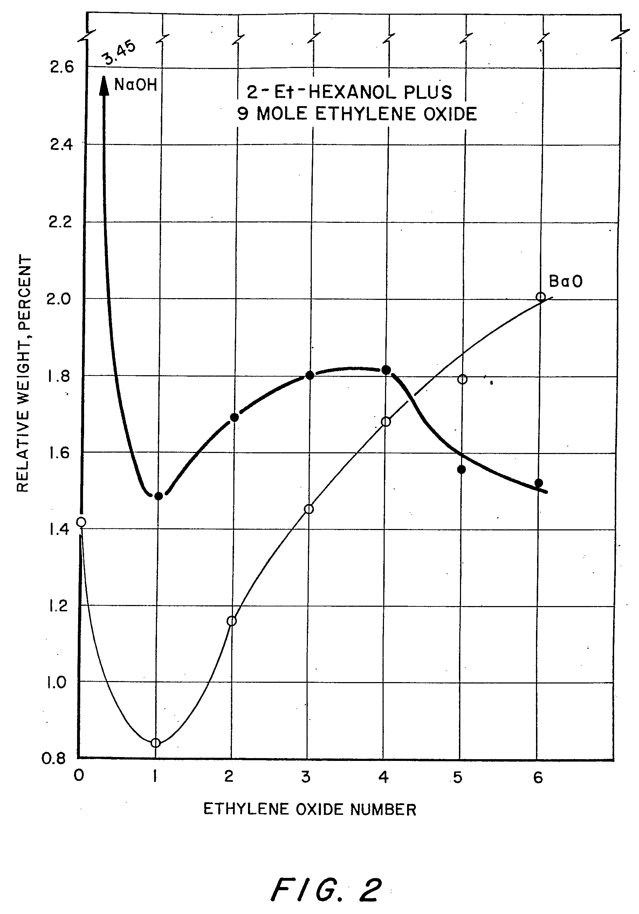

Figs. 1 and 2 compare the distributions obtained by barium oxide and sodium hydroxide catalyzed ethoxylation reactions according to the invention, as explained in detail in Examples 1 and 2.

While the instant invention is effective with all classes of alkanols, both primary, secondary,

2

tertiary, linear and branched, linear and branched primary alkanols are the most commonly used alcohols and are the preferred alcohols of the instant invention. Especially linear primary alcohols containing from 10 to 16 carbon atoms are preferred. Representative examples of such alcohols are those derived by hydrogenation of natural fats and oils, such as CO and TA alcohols, trademark of and sold by Procter and Gamble Co., such as CO-1214N alcohol, CO 1618 alcohol, and TA 1618 alcohol, and ADOL alcohols, trademark of and sold by Ashland Oil Co., such as ADOL 54 alcohol, ADOL 61 alcohol, ADOL 64 alcohol, ADOL 60 alcohol, and ADOL 66 alcohol. Alcohols produced by Ziegler chemistry can also be ethoxylated. Examples of these alcohols are ALFOL* alcohols, trademark of and sold by Continental Oil Co., such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols (hydroformylation) produced from olefins. Examples of such alcohols are NEODOL alcohols, trademark of and sold by Shell Oil Co., such as NEODOL 23 alcohol, NEODOL 25 alcohol, NEODOL 1418 alcohol; TERGITOL-L,* trademark of Union Carbide Corp. such as TERGITOL-L 125 alcohol; and isodecyl and tridecyl alcohols, sold by Exxon Corp., such as isodecyl alcohol and tridecyl alcohol. Guerbet alcohols can also be ethoxylated. Representative examples of these alcohols are STANDAMUL alcohols, trademark of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp.

Generally, useable alcohols include 1-decanol; 1-undecanol; 1-dodecanol; 1-tridecanol; 1-tetradecanol; 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 2-pentyl-1-tri-decanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol 2-tetradecanol; 3-tetradecanol; 4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecen-1-ol; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-3-tridecanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; 4-ethyl-3-decanol.

While pressure or lack of pressure is not a detriment to the process of the instant invention, normally a pressure of up to 7 kg/cm² can be used. Preferred pressures would be from 0.7 to 3.5 kg/cm². However, it must be realized that the reaction can be carried out in a vacuum or at pressures above 7 kg/cm² if desired. It is simply more convenient to carry out the reaction in the pressure range of from about atmospheric to 7 kg/cm².

The instant invention is carried out at temperatures of from 93°C to 260°C. However, for practical reasons, commercial operations will normally be carried out at temperatures in the range of from about 149°C to 204°C and the most preferred temperature is 177°C.

The reaction products of the described reaction can have any desired content of ethylene oxide but will normally range from 30 to 80% content of ethylene oxide (EO) based on weight. However, for most purposes the content of ethylene oxide will range from 40% to 70%. The amount of EO present in the reaction is not critical other than the minimum amount necessary to provide sufficient units to reach the mole adduct level desired for the alcohol present. Excess EO does not affect the reaction.

The catalyst used in the instant invention, which is barium oxide alone, barium metal, barium hydroxide, or a barium hydroxide hydrate, is a basic catalyst which provides a sharp distribution as to the mole adducts formed while reducing greatly the amount of unreacted free alcohols and undesirable by-products normally found in sharp distribution reactions. Barium oxide appears to be unique since tests carried out with oxides of calcium and magnesium failed to reveal any significant ethoxylation capacity.

Any of these barium compounds are effective in the process of the instant invention. When used, these catalysts can be used in any desired quantity. The larger the quantity used the more quickly the reaction goes to completion, although larger quantities do not appear to significantly alter the distribution obtained. However, for practical reasons, normally from 0.1 to 0.5 weight percent based upon the weight of the alcohol to be reacted will be present in the reactor. However, it must be very

*Registered Trade Mark.

**0 006 105**

clear that these limits can be varied substantially since the catalyst is effective at all levels and that catalyst concentration is simply a reaction rate modifier and not a reaction distribution modifier.

Such barium-containing catalysts include $BaO$, $Ba(OH)_2$ and $Ba(OH)_2 \cdot XH_2O$ wherein X represents the number of water molecules present. X is not a critical number.

Generally, treatment of alcohols with ethylene oxide yields a non-ionic detergent since hydrogen bonding to the numerous oxygen atoms makes the polyether end of the molecule water soluble. Alternatively, the ethoxylates can be converted into sulfates and used in the form of sodium salts.

The instant invention thus provides the production of highly efficient alcohol ethoxylates from primary, secondary, and tertiary branched chain and straight chain alkanols in a novel, highly unexpected manner. The ethoxylate products normally have from 4 to 20 carbon atoms. The reaction products are useful as a non-ionic surface active agent with high wetting power which are composed of mixtures of monoalkyl ethers of polyethylene glycol.

Thus in the preferred form of the instant invention, ethylene oxide is reacted with a branched chain or straight chain higher alkanol in the presence of barium oxide, barium hydroxide, a barium hydroxide hydrate or barium metal.

The invention is more concretely described with reference to the examples below, wherein all parts and percentages are by weight unless otherwise specified. The examples are provided to illustrate the instant invention and not to limit it.

Example 1

The ethoxylations were carried out in a stirred autoclave. Experimental conditions are summarized in Table 1. Ethylene oxide (EO) was added as a liquid against a constant nitrogen back pressure through a control valve. As the EO reacted with the alcohols present, the pressure in the reactor was reduced, and additional EO was added through the control valve until desired pressure was again obtained. Thus a constant pressure, self-adjusting reaction took place. Figure 1 compares the distributions obtained from barium oxide and sodium hydroxide catalyzed reactions. An examination of the figure will show the extremely sharp distribution produced by barium oxide as compared to sodium hydroxide.

TABLE 1
Ethoxylation of $C_{12}$ alcohol

| Catalyst | NaOH | BaO |
|---|---|---|
| ALFOL/g | 200.0 (1.07 mole) | 200.0 (1.07 mole) |
| EO/g | 426.0 (0.67 mole) | 426.0 (9.67 mole) |
| Cat./g | 0.2 | 0.3 |
| EO press./kg/cm² | 2.8 | 2.8 |
| Temp./°C | 177 | 204 |
| Reaction time/hr | 6.5 | 2.0 |

Example 2

Table 2 summarizes experimental conditions used in the ethoxylation of 2-ethylhexanol-1. Again reference is made to the reaction conditions of the table and to Figure 2 which shows the very narrow distribution obtained with barium oxide as compared to sodium hydroxide.

TABLE 2
Ethoxylation of Et-Hexanol-1

| Catalyst | NaOH | BaO |
|---|---|---|
| Alcohol/g | 75.0 (0.576 mole) | 75.0 (0.576 mole) |
| EO/g | 228.0 (5.18 mole) | 228.0 (5.18 mole) |
| Cat./g | 0.08 | 0.15 |
| EO press/kg/cm² | 3.5 | 3.5 |
| Temp./°C | 173 | 201 |
| Reaction time/hr | 4.5 | 2.0 |

In the figures, the EO number was determined using well-known gas chromatographic techniques.

Example 3

The base catalyzed ethoxylation of $C_{10}$ alcohols produced as straight chain primary alcohols of even carbon numbers using a modified Ziegler-Natta polymerization of ethylene (sold as ALFOL 10 alcohols, trademark of and produced by Continental Oil Company) was carried out under the reaction conditions described in Table 3.

4

TABLE 3
Ethoxylation of Alfol 10

| Catalyst | NaOH | Bao | $Ba(OH)_2 \cdot 8H_2O$ |
|---|---|---|---|
| Alcohol/g | 300 | 300 | 300 |
| EO/g | 251 | 251 | 251 |
| Cat./g | 0.3 | 1.0 | 2.0 |
| EO pressure/kg/cm$^2$ | 1.4 | 1.4 | 1.4 |
| Temp/°C | 204 | 204 | 204 |
| Reaction time/hr | 2.8 | 2.3 | 3.7 |

Relative weight percents of the ethylene oxide units adducted onto the alcohol are shown in Table 4.

TABLE 4
Relative weight percent

| EO unit | NaOH | BaO | $Ba(OH)_2 \cdot 8H_2O$ |
|---|---|---|---|
| 0 | 12.74 | 9.76 | 9.38 |
| 1 | 9.56 | 8.15 | 8.61 |
| 2 | 10.47 | 11.40 | 12.76 |
| 3 | 10.47 | 13.52 | 15.19 |
| 4 | 9.26 | 12.61 | 14.08 |
| 5 | 7.18 | 9.08 | 10.19 |
| 6 | 5.64 | 4.86 | 5.81 |
| 7 | 3.54 | 1.10 | 2.76 |
| 8 | 1.56 | — | 0.76 |

It has also been surprisingly discovered that the method of the instant invention is extremely well suited for ethoxylation of alcohols produced from olefins by hydroformylation/hydrogenation. Such alcohols have in the past presented difficulty when used as reactants for ethoxylation because of high concentration of unreacted alcohols.

However, the catalysts used in the instant invention produce an extremely good ethoxylate using these products.

Thus it is apparent by practicing the instant invention high mole adduct, ethoxylates of alcohols can be obtained in a very narrow, highly desirable distribution range while producing very low amounts of by-products and unreacted free alcohols.

Gas chromatographic analysis of the experiments described above showed the basic barium-containing catalysts used in the instant invention to be low in by-product and unreacted free alcohols. A comparison with NaOH showed BaO to favorably compare to the known basic catalyst. Both BaO and NaOH ethoxylation products contained less than 1 part per million (ppm) dioxane and less than 3 weight percent polyethylene glycol (based on total reaction product, and determined by solvent extraction). These basic catalysts produced reaction products far superior to those obtained in acid catalyzed ethoxylations, in which reaction product dioxane normally exceeds 1000 ppm and polyethylene glycol exceeds 3 weight percent.

The barium-containing catalysts used in the instant invention excell in the low levels of unreacted free alcohols in the reaction product. This superiority is clearly demonstrated in Figures 1 and 2, where the relative amounts of unreacted alcohol are graphically illustrated by referring to the locations of the respective graphs as they interset the zero ethylene oxide number axis. The distance between the point of intersection and the ordinate of the graph (0,0 value) indicates the free alcohols present. The unreacted free alcohols present in BaO catalyzed reaction products is only 30% of that present in NaOH catalyzed reaction product.

Although exemplified as a batch reaction, the catalyst used in the instant invention is also extremely well-suited to continuous reaction methods since the reaction products are of extremely desirable quality and quantity.

## Claims

1. A method for the ethoxylation of alcohols comprising contacting said alcohols with ethylene oxide in the presence of a catalyst at a temperature of from 93°C to 260°C, characterized in that the catalyst is barium oxide, barium hydroxide, hydrated barium hydroxide or barium metal.

2. A method as described in Claim 1 wherein the catalyst is barium oxide or hydrated barium hydroxide.

3. A method as described in Claim 2 wherein the alcohol contains from 4 to 20 carbon atoms.

4. A method as described in Claim 3 wherein the alcohol is a primary alcohol.

5. A method as described in Claim 4 wherein the alcohol is a product of a hydroformylation/hydrogenation reaction.

6. A method as described in Claim 5 wherein the reaction is carried out at a pressure up to 7 kg/cm².

7. A method as described in Claim 6 wherein the content of ethylene oxide in the ethoxylated product ranges from 30 weight percent to 80 weight percent.

8. A method as described in Claim 7 wherein the barium-containing catalyst is present in an amount from 0.1 to 0.5% by weight based upon the alcohol to be reacted.

9. A method as described in Claim 2 when carried out as a continuous reaction.

10. A method as described in Claim 7 wherein the alcohol is a linear primary alcohol containing from 10 to 16 carbon atoms, the ethylene oxide is present in such an amount that the reaction product contains 70 percent ethylene oxide, the temperature is 177°C, the pressure is 3.5 kg/cm² and the barium-containing catalyst is present in a concentration of from 0.1 to 0.5% based on the weight of the alcohol to be reacted.

## Patentansprüche

1. Verfahren zur Äthoxylierung von Alkohol, wobei man die Alkohole mit Äthylenoxid in Gegenwart eines Katalysators bei einer Temperatur von 93° bis 260°C in Kontakt bringt, dadurch gekennzeichnet, daß der Katalysator Bariumoxid, Bariumhydroxid, hydratisiertes Bariumhydroxid oder Bariummetall ist.

2. Verfahren nach Anspruch 1, wobei der Katalysator Bariumoxid oder hydratisiertes Bariumoxid ist.

3. Verfahren nach Anspruch 2, wobei der Alkohol 4 bis 20 Kohlenstoffatome enthält.

4. Verfahren nach Anspruch 3, wobei der Alkohol ein primärer Alkohol ist.

5. Verfahren nach Anspruch 4, wobei der Alkohol ein Produkt einer Hydroformylierungs-/Hydrierungsreaktion ist.

6. Verfahren nach Anspruch 5, wobei die Reaktion bei einem Druck bis zu 7 kg/cm² durchgeführt wird.

7. Verfahren nach Anspruch 6, wobei der Äthylenoxidgehalt in dem äthoxylierten Produkt im Bereich von 30 bis 80 Gew.% liegt.

8. Verfahren nach Anspruch 7, wobei der bariumhaltige Katalysator in einer Menge von 0,1 bis 0,5 Gew.%, bezogen auf den umzusetzenden Alkohol vorliegt.

9. Verfahren nach Anspruch 2, durchgeführt als eine kontinuierliche Reaktion.

10. Verfahren nach Anspruch 7, wobei der Alkohol ein kettenförmiger primärer Alkohol mit 10 bis 16 Kohlenstoffatomen ist, das Äthylenoxid in einer solchen Menge vorliegt, daß das Reaktionsprodukt 70% Äthylenoxid enthält, die Temperatur 177°C beträgt, der Druck 3,5 kg/cm² beträgt und der bariumhaltige Katalysator in einer Konzentration von 0,1 bis 0,5 %, bezogen auf das Gewicht des umzusetzenden Alkohols, anwesend ist.

## Revendications

1. Un procédé pour l'éthoxylation d'alcools, selon lequel on met les alcools en contact avec l'oxyde d'éthylène en présence d'un catalyseur à une température comprise entre 93°C et 260°C, caractérisé en ce que le catalyseur est de l'oxyde de baryum, de l'hydroxyde de baryum, de l'hydroxyde de baryum hydraté ou du baryum métallique.

2. Un procédé tel que décrit dans la revendication 1, dans lequel le catalyseur est de l'oxyde de baryum ou de l'hydroxyde de baryum hydraté.

3. Un procédé tel que décrit dans la revendication 2, dans lequel l'alcool contient de 4 à 20 atomes de carbone.

4. Un procédé tel que décrit dans la revendication 3, dans lequel l'alcool est un alcool primaire.

5. Un procédé tel que décrit dans la revendication 4, dans lequel l'alcool est un produit d'une réaction d'hydroformylation/hydrogenation.

6. Un procédé tel que décrit dans la revendication 5, dans lequel la réaction est conduite à une pression allant jusqu'à 7 kg/cm².

7. Un procédé tel que décrit dans la revendication 6, dans lequel la teneur en oxyde d'éthylène dans le produit éthoxylé est comprise entre 30 % en poids et 80 % en poids.

8. Un procédé tel que décrit dans la revendication 7, dans lequel le catalyseur contenant du baryum est présent à raison de 0,1 à 0,5 % en poids par rapport à l'alcool devant réagir.

9. Un procédé tel que décrit dans la revendication 2 quand il est mis en oeuvre sous la forme d'une réaction continue.

10. Un procédé tel que décrit dans la revendication 7, dans lequel l'alcool est un alcool primaire linéaire contenant de 10 à 16 atomes de carbone, l'oxyde d'éthylène est présent en quantité telle que le produit de réaction contienne 70 pour cent d'oxyde d'éthylène, la température est de 177°C, la pression est de 3,5 kg/cm² et le catalyseur contenant du baryum est présent à une concentration comprise entre 0,1 et 0,5 % par rapport au poids de l'alcool devant réagir.

FIG. 1

FIG. 2